# EUROPEAN PATENT SPECIFICATION

(11) **EP 1 618 916 B1**
(45) Date of publication and mention of the grant of the patent: **09.04.2008**
(21) Application number: 04103515.5
(22) Date of filing: 22.07.2004
(51) Int. Cl.: A61M 39/28, A61M 5/168, F16K 15/14

(54) **Automatic locking valve for medication injection**
Automatisches Verschlußventil für eine medizinische Infusion
Vanne à fermeture automatique pour perfusions médicales

(43) Date of publication of application: 25.01.2006
(73) Proprietor: Woo Young Medical Co., Ltd., Paju-City, Kyunggi-Do (KR)
(72) Inventor: Lee, Young Gyu, Sungbuk-Gu, Seoul (KR); Kim, Ki Woon, Ilsan-Gu, Goyang-City, Kyunggi-Do (KR)
(74) Representative: Gervasi, Gemma

(56) References cited:
- FR-A- 2 682 603
- US-A- 3 827 439
- US-A- 4 398 542
- US-A- 5 396 925
- US-A- 6 077 055
- US-A1- 2002 151 838

## Description

### BACKGROUND OF THE INVENTION

### Field of the Invention

The present invention relates to an automatic locking valve for medication injection, and more particularly to an automatic locking valve for medication injection, which is provided with a blocking sensor for sensing blockage of a tube and with automatic locking means for performing an operation corresponding to a mounting state of a door such that a dose of medication can be administered only under an installation condition in which the door is completely closed after the tube is installed, so that the dose of medication can be safely administered to a patient.

### Description of the Related Art

As is well known in the prior art, in order to administer a dose of medication prescribed by a doctor to a patient, after the dose of medication is placed at a higher position than an injecting position at the patient, a valve with an on/off part of a torsional screw-like shape therein should be adjusted in accordance with the experience of a nurse performing injection of the predetermined dose to the patient.

When the patent is administered with the dose of medication, such as an analgesic, there often come intermittent convolutions or pains, forcing a temporary increase of the dose of injection. With regard to this, since the dose should be administered according to a prescription of the doctor, it is very dangerous for the patient to adjust his or her own dose of injection. However, under the present circumstances of hospitals, a prompt adjustment for the dose of injection corresponding to the intermittent pains of the patient is very difficult for medical staffs and causes very serious consumption of time for them.

For a nurse inexperienced in adjusting the dose of injection, it is very difficult to adjust a dose of injection an hour such that the prescribed dose is administered with accuracy. Further, in case of a patient with a special disease, such as acute cardiac paralysis, a requirement of being administered only when the patient suffers an attacks forces the patient to always carry the medication for the disease and a requirement of administrating an accurate dose of medication requires great deal of skill in administration.

Even for an expert in administration, there is always a danger of unintentional excessive administration of the medication. Further, when extraneous substances or air are contained in the medication or when something presses a tube, it poses a serious threat to the patient.

Although an automatic injector for automatically injecting a predetermined dose of medication has been recently developed, it simply adopts a compulsory injection system for injecting the medication using a water pump and does not have separate sensors for sensing the dose injected, thereby causing a very high possibility of safety accident. Further, when a door is being opened, an excessive dose of medication can be injected at one time, resulting in potentially life-threatening situation.

US-A-2002/0151838 discloses a feeding set adaptor for delivering solutions comprising a sample cell for determining the presence of air within an infusion set, and an anti-freeflow device for selectively preventing freeflow through the infusion set.

### SUMMARY OF THE INVENTION

The present invention has been made to solve the above problems, and it is an object of the present invention to provide an automatic locking valve for medication injection, which is provided with a blocking sensor for sensing blockage of a tube and with automatic locking means for performing an operation corresponding to a mounting state of a door such that a dose of medication can be administered only under an installation condition in which the door is completely closed after the tube is installed, so that the dose of medication can be safely administered to a patient.

In accordance with an aspect of the present invention, the above and other objects can be accomplished by the provision of an automatic dose administrating apparatus having the features of claim 1.

Preferably, the automatic locking valve comprises: a valve housing for defining a space therein, the valve housing having input and output ports for allowing the medication to flow in and out; an on/off member of a spool-like shape, the on/off member having a diameter smallest at the center thereof and being tapered to linearly increase toward upper and lower portions of the on/off member so that the on/off valve moves up and down depending on the closed state of the door; and a membrane attached to the space of the valve housing such that the membrane expands toward a lower portion of the space due to elastic properties of the membrane when the tube is blocked.

More preferably, the space of the valve housing has a shape tapered for a diameter of the space to increase from the center to an upper portion and to increase from the center to a lower portion in order to correspond to the shape of the on/off member; and the lower portion of the space has an increased inner diameter, so that when the on/off member is pressed down, the on/off member defines a fluid gap along with an inner circumferential surface of the valve housing.

Preferably, the valve housing further comprises a blocking sensor for generating a "High" signal with liquid or metal at a predetermined portion of the membrane and for sensing blockage of the tube when the membrane swells to expand toward the blocking sensor.

### BRIEF DESCRIPTION OF THE DRAWINGS

The foregoing and other objects and features of the present invention will be more clearly understood from the following detailed description taken in conjunction with the accompanying drawings, in which:
Figs. 1a and 1b are side sectional views showing construction and operation of an automatic locking valve for medication injection in accordance with an embodiment of the present invention;
Fig. 2 is a side sectional view showing operation of a membrane provided in the automatic locking valve for injecting a dose of medication in accordance with the embodiment of the present invention;
Fig. 3 is a perspective view of an appearance of an automatic dose administrating apparatus in accordance with an embodiment of the present invention;
Fig. 4 is a partial perspective view showing, in detail, the construction of the automatic dose administrating apparatus;and
Fig. 5 is a perspective view of another embodiment of the automatic dose administrating apparatus.

### DESCRIPTION OF THE PREFERRED EMBODIMENTS

Preferred embodiments of the present invention will now be described in detail with reference to the accompanying drawings.

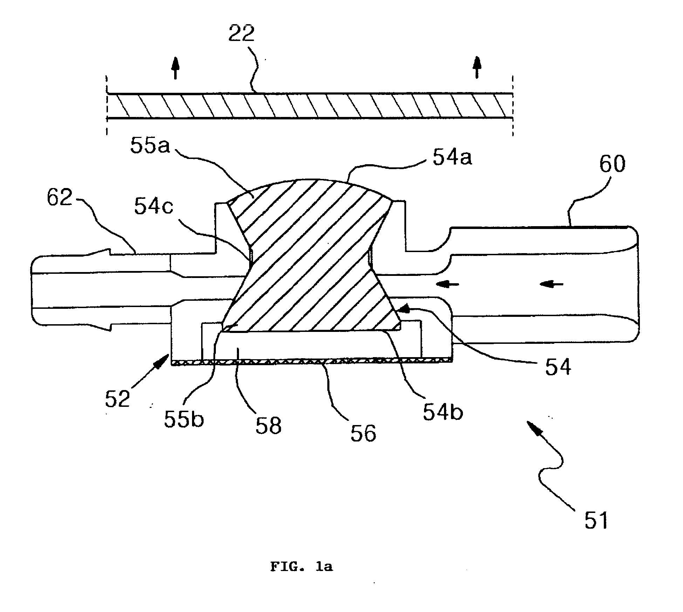

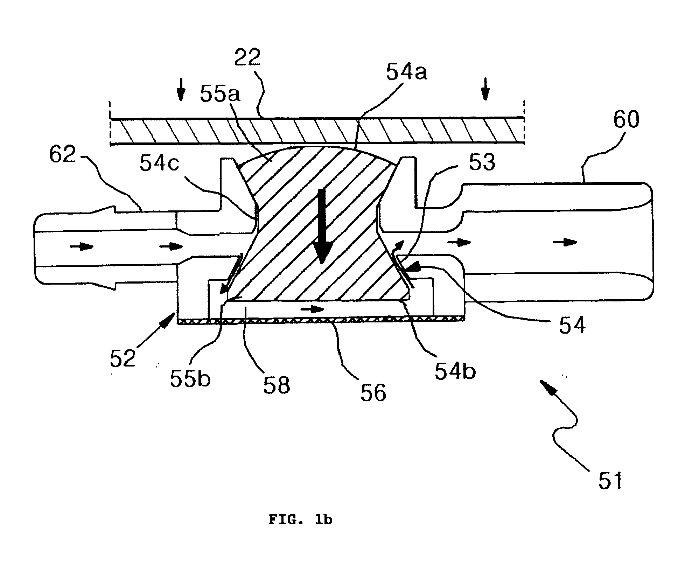

Figs. 1a and 1b are side sectional views showing construction and operation of an automatic locking valve for medication injection in accordance with an embodiment of the present invention.

An automatic locking valve for medication injection 51 in accordance with the embodiment of the present invention is provided with a blocking sensor for sensing blockage of a tube and with automatic locking means for performing an operation corresponding to a mounting state of a door such that a dose of medication can be administered only under an installation condition in which the door is completely closed after the tube is installed, so that a patient can safely administer the dose of medication for oneself.

The automatic locking valve 51 of the present invention comprises a valve housing 52, which defines a space 58 therein and has input and output ports 60 and 62 connected with a tube (not shown), respectively, for allowing the medication to flow in and out.

The valve housing 52 defines the space 58 therein for receiving an on/off member 54 therein. The space 58 has a shape tapered for a diameter of the space to increase from the center to an upper portion and to increase from the center to a lower portion in order to correspond to the shape of the on/off member 54.

The on/off member 54 generally has a cylindrical shape. Specifically, like a spool, the on/off member 54 has a diameter smallest at the center thereof and tapered for the diameter of the on/off member to linearly increase toward upper and lower portions thereof, respectively. Here, the upper portion of the on/off member 54 acts as an on/off operation portion 55a and the lower portion thereof acts as an on/off portion 55b for controlling the medication directly flowing through the valve.

Specifically, with a cross-section of a circular shape in the horizontal plane, the on/off member 54 has an outer diameter increased from the center to the upper and lower portions, respectively, thereby forming the spool-like shape. The on/off member 54 has the top portion 54a curved convexly at the center thereof and the bottom portion 54b flattened to be parallel with the ground.

The on/off member 54 is elastic. Thus, when the on/off member 54 is pushed into the space defined by the upper portion of the valve housing 52, the bottom portion and the outer circumferential portion thereof are compressed inwardly toward the inner center of the on/off member 54, so that it is gradually pushed into the space 58 of the valve housing 52, whereby the on/off member 54 is finally engaged with the valve housing 52.

When the on/off member 54 is installed in the valve housing 52, the flow of the medication through the space 58 of the valve housing 52 is controlled by the inlet and outlet ports 60 and 62, depending on pressure to be applied to the top surface of the on/off member 54. Specifically, with the on/off member 54 installed in the valve housing 52, the on/off member moves up and down inside the valve housing 62 by a predetermined distance. Thus, when the on/off operation member 55a of the on/off member 54 maintains close contact with the inner circumferential surface of the valve housing 52, there is no fluid gap allowing the medication to flow between them. However, when the on/off portion 55b at the lower portion thereof defines a fluid gap 53 along with the inner circumferential surface of the valve housing 52, the medication can flow into the lower portion of the space 58 through the fluid gap 53.

Thus, depending on whether the on/off member 54 is pushed down or not, the medication passed through the outlet port 60 flows or does not flows toward the inlet port 62 through the space 58.

Specifically, if the on/off member 54 is pushed down, as shown in Fig. 1b, the fluid gap 53 is opened so that the medication passing through the outlet port 60 can flow out via the space 58. If the on/off member 54 is not pushed down, as shown in Fig. 1a, the fluid gap 53 is closed so that the medication passing through the outlet port 60 cannot flow out because the medication passing through the outlet port 60 cannot flow into the space 58.

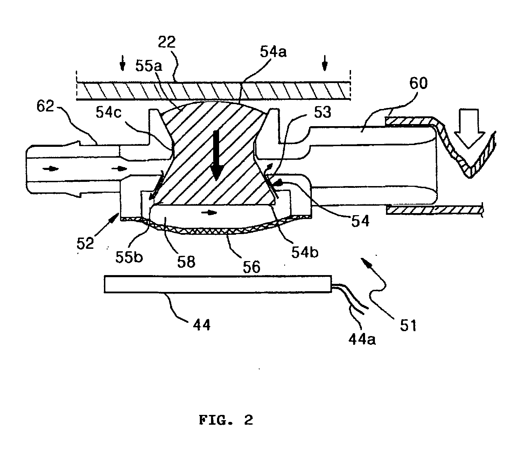

Fig. 2 is a side sectional view showing operation of a membrane provided in the automatic locking valve for injecting a dose of medication in accordance with the embodiment of the present invention.

Referring to Fig. 2, the automatic locking valve 51 of the invention further comprises a membrane 56 at the lower portion of the space 58, of which the periphery is attached around the edge of a lower surface of the space 58 defined by the membrane 56.

The membrane 56 is made of a fine film and in spite of being elastic, does not expand when the medication flows. When the middle of a valve (not shown) connected with the inlet port 62 is folded or pressed down by something so that the medication cannot normally flow through the valve, the membrane 56 expands convexly in the direction of the lower portion for the pressure of accumulated medication in the space 58.

Further, as shown in Fig. 2, the membrane 56 is provided with, at its predetermined lower portion, a blocking sensor 44 for generating a "High" signal with liquid or metal. Thus, when the medication does not regularly flow, for example, due to the tube being pressed down on by something, the membrane 56 swells to expand toward the blocking sensor 44, thereby sensing the blockage of the tube.

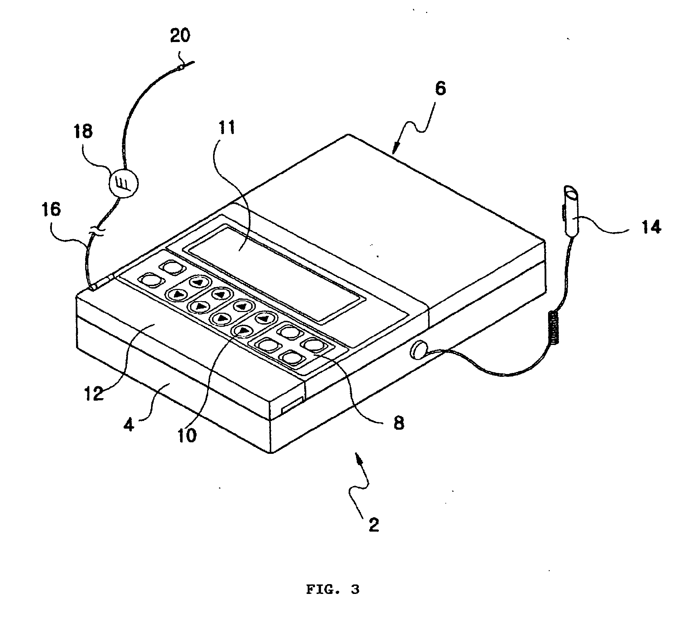

Fig. 3 is a perspective view of an appearance of an automatic dose administrating apparatus applied with the automatic locking valve 51 for medication injection according to the embodiment of the present invention.

Referring to Fig. 3, the automatic dose administrating apparatus 2 according to an embodiment of the present invention is provided with the automatic locking valve for sensing a mounting state of a door and a blocking sensor for sensing blockage of a tube, such that the dose of medication can be administered under a condition of completed installation with the door closed above the tube mounted in the apparatus, whereby a patient can safely administer his or her own dose of medication.

The automatic lock valve for medication administration of the invention can be applied to the automatic dose administrating apparatus 2, as shown in Fig. 3. The automatic dose administrating apparatus 2 is provided with a housing 4 divided into three compartments. Among the compartments, the uppermost compartment defines a medication storage chamber 6 storing a medication pack therein, and the middle compartment defines a control chamber 8 having an LCD 11 for displaying the installation state.

Under the control chamber 8, the lowermost compartment defines a driving room 12 for controlling injection of the medication while driving a discharge of the medication through tube 16 by pumping the medication.

As the housing 4 is provided with a ball-loose switch 14 at a predetermined part of the side thereof, the user can temporarily increases the dose of injection by operating the ball-loose switch 14. The tube 16 is provided with a needle 20 at one end thereof and also provided with an air-filter 18 at the middle of the tube 16 extending from the housing 4 to the needle 20. The air-filter 20 discharges air contained in the medication flowing through the tube 16 after filtering air. The control part 8 is provided with a button part 10 on the surface thereof, which can set a various settings by operation of its keys.

Using the automatic dose administrating apparatus 2 installed with the sensors as described above, the patient can be very conveniently administered with a predetermined dose of medication without any restriction by time or place while always carrying it. Further, due to the sensor being organically associated with each other to ensure safety of the patient during administration, the patient can control the administration of the medication and does not need an additional nurse in a daily life.

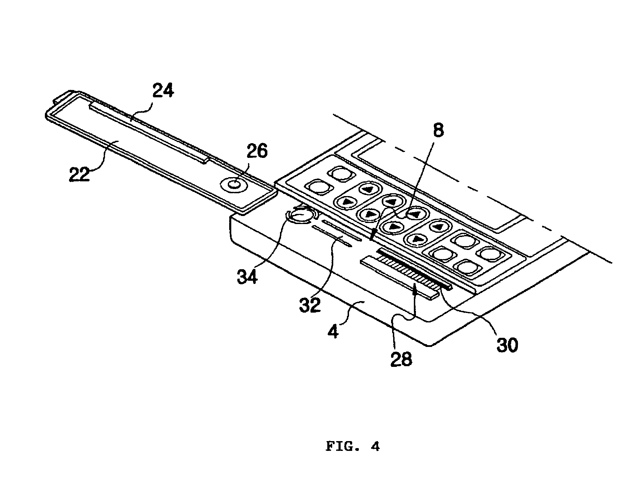

Fig. 4 is a partial perspective view showing in detail the construction of the automatic dose administrating apparatus with the automatic locking valve 51 for medication injection according to the embodiment of the present invention applied to it.

Referring to Fig. 4, at lowermost portion of the housing 4, the automatic dose administrating apparatus 2 is provided with the driving room 12 for generating driving force to inject the medication with an application of a signal for controlling the injection of the medication. The driving room 12 is provided with a door 22 hinged at the top of the driving room 12. At the bottom of the door 22, the door 22 is provided with a supporting bar 24 for pressing the tube 16 and with a pressing protrusion 26 for making a press in order to maintain a check valve of the tube 16 in an open state.

The driving room 12 is provided with a pressure pump 28 therein, comprising a motor (not shown) for generating a rotational driving force of a predetermined revolutions per minute with an electric control and a plurality of longitudinally connected pressing pieces 30 which move up and down with supply of the rotational driving force.

The pressing piece 30 sinusoidally moves up and down by a rotational force of a cam (not shown). Thus, the pressing piece 30 presses the bottom of a main tube (not shown) to the sinuous shape, causing the medication in the tube 16 to flow in one direction.

On the top of the housing 4 to which the door 22 of the driving room 12 is connected, the housing 4 is formed with a guiding groove 32 for guiding the tube 16 in the state of mounting the tube 16 in the housing, and with a check valve receiving groove 34 at the side of the guiding groove 32.

If the door 22 is closed after the automatic locking valve 51 is inserted into the driving room with the medication pack (not shown) received in the medication storage chamber 6, the bottom of the door 12 presses the on/off member 54 of the automatic locking valve 51, generating a flow passage of the medication with the fluid gap opened. Meanwhile, if the door 22 is opened, the on/off member 54 returns back to the upper portion thereof due to elasticity, closing the flow passage of the medication with the fluid gap closed. Further, if the medication does not regularly flow, for example, due to the tube pressed by something in the middle thereof, the membrane 56 swells to expand toward the blocking sensor 44, so that the blocking sensor 44 can sense the blockage of the tube, whereby the patient can be safely administered with medication for himself or herself.

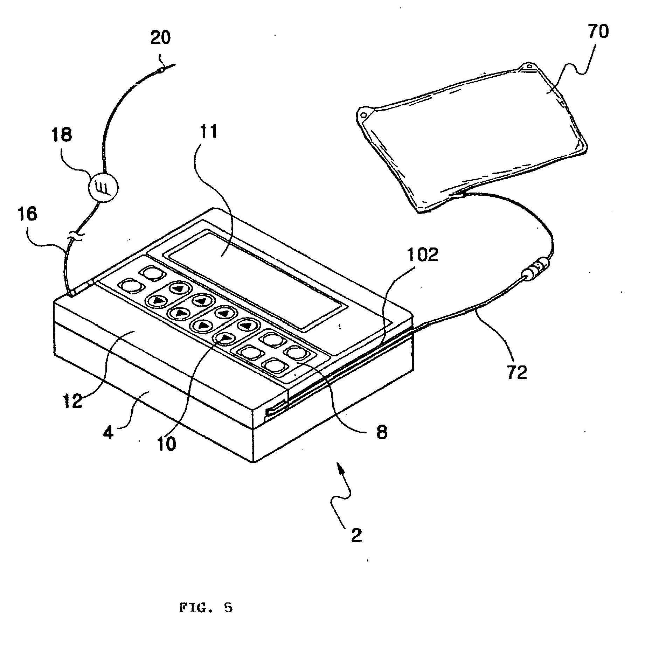

Fig. 5 is a perspective view of an automatic dose administrating apparatus according to another embodiment of the present invention, in which similar components to those of the automatic dose administrating apparatus of Fig. 3 are indicated by the same reference numerals as those of Fig. 3.

Referring to Fig. 5, an automatic dose administrating apparatus 2 with the automatic locking valve 51 for medication injection of the present invention applied to it may be provided with a connecting tube 72 directly connected to a special medication pack 70, instead of the medication storage chamber 6 as shown in Fig. 3.

In this case, the housing 4 of the automatic dose administrating apparatus 2 is preferably provided with a slot 102 at an outer side thereof in the lengthwise direction for leading the connecting tube 72. Thus, regardless of the type of medication pack, the medication pack 70 can be connected to the automatic dose administrating apparatus 2 using the connecting tube 72, and a predetermined dose of medication can be administered to the patient from the inside of the automatic dose administrating apparatus 2.

As is apparent from the description, in accordance with the present invention, the automatic locking valve for medication injection is provided with an automatic locking valve for sensing a mounting state of a door and a blocking sensor for sensing blockage of a tube, such that a dose of medication can be administered under a condition of completed installation with the door closed above the tube mounted in the automatic locking valve, thereby providing safety and accuracy when administering the dose to a patient.

It should be understood that the embodiments and the accompanying drawings as described above have been described for illustrative purposes and the present invention is limited by the following claims. Further, those skilled in the art will appreciate that various modifications, additions and substitutions are allowed without departing from the scope of the invention as set forth in the accompanying claims.

## Claims

1. An automatic dose administrating apparatus (2) comprising:
a housing (4) comprising a driving room (12) for controlling the injection state of a medication from a medication pack, the driving room being provided with a pressure pump for driving discharge of the medication by pumping the medication;
a tube (16) for transferring the medication discharged from the medication pack to a needle, the tube extending from an inner part of the driving room (12) to an outer part thereof and the medication pumped by the pressure pump being discharged through the tube (16),
wherein the automatic dose administrating apparatus (2) further comprises an automatic locking valve (51) which is mounted in the driving room (12) such that the automatic locking valve (51) is connected, at one side, to the tube (16) connected to the medication pack and at the other side, to the tube connected to the needle, such that the automatic locking valve (51) controls flow of the medication therein depending on a closed state of a door of the driving room (12),
**characterised in that**
the housing (4) supports outer portions of three compartments further comprising a medication container (6) for storing the medication pack therein,
a control chamber (8) having a displaying unit for displaying an installation state,
and **in that** the automatic locking valve (51) comprises a valve housing (52) for defining a space therein, the valve housing having input and output ports (60, 62) for allowing the medication to flow in and out; an on/off member of a spool-like shape (54), the on/off member having a diameter smallest at the centre thereof and being tapered to linearly increase toward upper and lower portions of the on/off member so that the on/off valve moves up and down depending on the closed state of the door; and a membrane (56) attached to the space of the valve housing such that the membrane expands toward a lower portion of the space due to elastic properties of the membrane when the tube is blocked.

2. The automatic dose administrating apparatus as set forth in claim 1, wherein the space of the valve housing (52) has a shape tapered for a diameter of the space to increase from the centre to an upper portion and to increase from the centre to a lower portion in order to correspond to the shape of the on/off member (54); and the lower portion of the space has an increased inner diameter, so that when the on/off member is pressed down, the on/off member defines a fluid gap along with an inner circumferential surface of the valve housing.

3. The automatic dose administrating apparatus as set forth in claim 1, wherein the valve housing (52) further comprises a blocking sensor (44) for generating a "High" signal with liquid or metal at a predetermined portion of the membrane and for sensing a blocked state of the tube when the membrane swells to expand toward the blocking sensor (44).

## Patentansprüche

1. Automatische Dosisverabreichungsvorrichtung (2), die umfasst:
ein Gehäuse (4), das einen Antriebsraum (12) zum Steuern des Injektionszustands einer Medikation von einer Medikationspackung umfasst, wobei der Antriebsraum mit einer Druckpumpe zum Antreiben eines Ausstoßes der Medikation durch Pumpen der Medikation versehen ist;
einen Schlauch (16) zum Übertragen der ausgestoßenen Medikation von der Medikationspackung zu einer Nadel, wobei sich der Schlauch von einem inneren Teil des Antriebsraums (12) zu einem äußeren Teil davon erstreckt und die durch die Druckpumpe gepumpte Medikation durch den Schlauch (16) hindurch ausgestoßen wird,
wobei die automatische Dosisverabreichungsvorrichtung (2) ferner ein automatisches Sperrventil (51) umfasst, das derart in dem Antriebsraum (12) angebracht ist, dass das automatische Sperrventil (51) an einer Seite mit dem Schlauch (16), welcher mit der Medikationspackung verbunden ist, und an der anderen Seite mit dem Schlauch verbunden ist, welcher mit der Nadel verbunden ist, so dass das automatische Sperrventil (51) das Strömen der Medikation darin in Abhängigkeit von einem geschlossenen Zustand einer Tür des Antriebsraums (12) steuert,
**dadurch gekennzeichnet, dass**
das Gehäuse (4) äußere Abschnitte von drei Abteilen trägt, die ferner einen Medikationsbehälter (6) zum Unterbringen der Medikationspackung darin und
eine Steuerkammer (8) umfassen, welche eine Anzeigeeinheit zum Anzeigen eines Installationszustands aufweist,
und **dadurch**, dass das automatische Sperrventil (51) umfasst: ein Ventilgehäuse (52) zum Definieren eines Raums darin, wobei das Ventilgehäuse einen Einlass- und einen Auslasskanal (60, 62) aufweist, um es der Medikation zu ermöglichen, ein- und auszuströmen; ein Ein/Aus-Element von spulenähnlicher Form (54), wobei das Ein/Aus-Element einen Durchmesser aufweist, der an dessen Zentrum am kleinsten ist und konisch ist, um linear in Richtung des oberen und unteren Abschnitts des Ein/Aus-Elements anzusteigen, so dass sich das Ein/Aus-Ventil in Abhängigkeit von dem geschlossenen Zustand der Tür auf und ab bewegt; und eine Membran (56), die an dem Raum des Ventilgehäuses befestigt ist, so dass sich die Membran aufgrund der elastischen Eigenschaften der Membran in Richtung eines unteren Abschnitts des Raums ausdehnt, wenn der Schlauch blockiert ist.

2. Automatische Dosisverabreichungsvorrichtung nach Anspruch 1, wobei der Raum des Ventilgehäuses (52) eine konische Form aufweist, so dass ein Durchmesser des Raums von dem Zentrum zu einem oberen Abschnitt ansteigt und von dem Zentrum zu einem unteren Abschnitt ansteigt, um mit der Form des Ein/Aus-Elements (54) übereinzustimmen; und dass der untere Abschnitt des Raums einen vergrößerten Innendurchmesser aufweist, so dass das Ein/Aus-Element, wenn das Ein/Aus-Element heruntergedrückt wird, einen Fluidspalt entlang einer inneren umlaufenden Oberfläche des Ventilgehäuses definiert.

3. Automatische Dosisverabreichungsvorrichtung nach Anspruch 1, wobei das Ventilgehäuse (52) ferner einen Blockiersensor (44) zum Erzeugen eines "Hoch"-Signals mit Flüssigkeit oder Metall an einem vorbestimmten Abschnitt der Membran und zum Erfassen eines blockierten Zustands des Schlauchs umfasst, wenn sich die Membran ausbaucht, um sich in Richtung des Blockiersensors (44) auszudehnen.

## Revendications

1. Appareil d'administration de dose automatique (2) comprenant :
un boîtier (4) comprenant une pièce d'entraînement (12) pour commander l'état d'injection d'un médicament d'un paquet de médicament, la pièce d'entraînement présentant une pompe de pression pour entraîner l'évacuation du médicament en pompant le médicament ;
un tube (16) pour transférer le médicament évacué du paquet de médicament à une aiguille, le tube s'étendant depuis une partie interne de la pièce d'entraînement (12) à une partie externe de celle-ci, et le médicament pompé par le pompe de pression étant évacué à travers le tube (16),
où l'appareil d'administration de dose automatique (2) comprend en outre une vanne de verrouillage automatique (51) qui est installée dans la pièce d'entraînement (12) de telle sorte que la vanne de verrouillage automatique (51) est reliée, à un côté, au tube (16) relié au paquet de médicament et à l'autre côté, au tube relié à l'aiguille de sorte que la vanne de verrouillage automatique (51) commande l'écoulement du médicament dans celle-ci en fonction d'un état fermé d'une porte de la pièce d'entraînement (12), **caractérisé en ce que**
le boîtier (4) supporte des portions extérieures de trois compartiments comprenant en outre un récipient de médicament (6) pour stocker le paquet de médicament dans celui-ci,
une chambre de commande (8) comportant une unité d'affichage pour afficher un état d'installation, et **en ce que** la vanne de verrouillage automatique (51) comprend un boîtier de vanne (52) pour définir un espace dans celui-ci, le boîtier de vanne ayant des orifices d'entrée et de sortie (60, 62) pour permettre au médicament de s'écouler vers l'intérieur et vers l'extérieur ; un élément marche/arrêt d'une forme de tiroir (54), l'élément marche/arrêt ayant un diamètre qui est le plus petit à son centre et diminuant pour augmenter linéairement vers les portions supérieure et inférieure de l'élément marche/arrêt de sorte que la vanne marche/arrêt monte et descend en fonction de l'état fermé de la porte ; et une membrane (56) fixée à l'espace du boîtier de vanne de telle sorte que la membrane s'expanse vers une portion inférieure de l'espace à cause des propriétés élastiques de la membrane lorsque le tube est bloqué.

2. Appareil d'administration de dose automatique selon la revendication 1, où l'espace du boîtier de vanne (52) a une forme diminuée pour qu'un diamètre de l'espace augmente du centre vers une portion supérieure et augmente du centre vers une portion inférieure pour correspondre à la forme de l'élément marche/arrêt (54) ; et la portion inférieure de l'espace présente un diamètre intérieur plus grand de sorte que lorsque l'élément marche/arrêt est enfoncé, l'élément marche/arrêt définit un espace de fluide le long d'une surface circonférentielle intérieure du boîtier de vanne.

3. Appareil d'administration de dose automatique selon la revendication 1, où le boîtier de vanne (52) comprend en outre un capteur de blocage (44) pour produire un signal "haut" avec du liquide ou du métal à une portion prédéterminée de la membrane et pour détecter un état bloqué du tube lorsque la membrane gonfle en s'expansant vers le capteur de blocage (44).
